# FASCICULE DE BREVET EUROPEEN

(11) **EP 1 745 089 B1**
(45) Date de publication et mention de la délivrance du brevet: **27.06.2012**
(21) Numéro de dépôt: 05762334.0
(22) Date de dépôt: 22.04.2005
(51) Int. Cl.: C08J 3/075, C08J 3/00, C08G 63/00, C08L 67/00

(54) **GEL CONCENTRE EN POLYESTER SULFONIQUE RAMIFIE ET PROCEDE DE PREPARATION DE CE GEL**
GEL MIT EINER HOHEN KONZENTRATION VON VERZWEIGTEM SULPHOPOLYESTER UND VERFAHREN ZU DESSEN HERSTELLUNG
GEL WITH A HIGH CONCENTRATION OF BRANCHED SULPHONIC POLYESTER AND METHOD FOR PREPARING SAME

(30) Priorité: 23.04.2004 FR 0450775; 18.05.2004 US 571916 P
(43) Date de publication de la demande: 24.01.2007
(73) Titulaire: L'Oréal, 75008 Paris (FR)
(72) Inventeur: GAWTREY, Jonathan, F-92100 Boulogne (FR)
(74) Mandataire: Bourdeau, Françoise
(86) Numéro de dépôt international: PCT/FR2005/001003
(87) Numéro de publication internationale: WO 2005/105899

(56) Documents cités:
- EP-A- 0 966 946
- FR-A- 2 760 360
- FR-A- 2 760 634
- GAONA O ET AL: "Preparation and hydrolytic degradation of sulfonated poly(ethylene terephthalate) copolymers" POLYMER, ELSEVIER SCIENCE PUBLISHERS B.V, GB, vol. 44, no. 24, novembre 2003 (2003-11), pages 7281-7289, XP004467294 ISSN: 0032-3861
- PATENT ABSTRACTS OF JAPAN vol. 1995, no. 10, 30 novembre 1995 (1995-11-30) & JP 07 187971 A (KAO CORP), 25 juillet 1995 (1995-07-25)

## Description

L'invention a pour objet une composition cosmétique se présentant sous la forme de gel fortement concentré en polyester sulfonique ramifié. Elle vise également le procédé de fabrication de ce gel ainsi que son utilisation pour la réalisation de compositions cosmétiques ayant une forme galénique quelconque.

Au sens de la présente invention, on entend par « *gel* », une composition initialement liquide, épaissie grâce à un agent ayant des propriétés épaississantes et/ou gélifiantes, ayant in fine une viscosité minimale de 160 cps, de préférence 250 cps à 25°C (viscosimètre Rhéomat 180 - mobile 2 - lecture après 30s) et une viscosité maximale de 10⁶ poises, de préférence de 10⁵ poises.

Il est connu l'utilisation de polyesters sulfoniques ramifiés en cosmétique. Dans le domaine du soin de la peau, on peut citer la demande de brevet déposée par la Demanderesse sous le numéro FR0300569, qui décrit des compositions moussantes contenant des polyesters sulfoniques ramifiés, la concentration en poids en polyester ramifié allant avantageusement entre 20 et 35% en poids par rapport au poids total de la composition.

En cosmétique capillaire, on peut citer le document FR 2 833 491, qui concerne l'utilisation des polyesters sulfoniques ramifiés dans des laques à l'eau. On peut aussi mentionner les documents EPO 966 946, WO98/38969, WO99/63955, relatifs à des compositions comprenant des polyesters sulfoniques ramifiés à des concentrations en poids allant de 0,1 à 40 % par rapport au poids total de la composition.

Ces documents indiquent, en toutes généralités, qu'il est possible de mettre en oeuvre les polyesters sulfoniques dans cette large gamme de concentrations, pour obtenir indifféremment des sprays, des mousses, des gels ou des lotions. Toutefois, les exemples ne donnent aucune composition contenant ces polyesters à forte concentration et se présentant sous la forme de gel.

Par ailleurs, on recherche aussi pour la peau, notamment du visage humain, et les phanères en particulier des cils et des sourcils, des produits ayant des propriétés de soin, de protection et/ou de conditionnement. De tels produits peuvent être des fonds de teint, des rouges à lèvres, des crèmes ou des gels pour le corps ou le visage, des ombres à paupières, des eye-liners, des mascaras, des blush.

En outre, que se soit pour la chevelure ou la peau, on recherche encore des compositions cosmétiques aptes à former sur ces matières kératiniques un film souple, qui suive les mouvements de la peau ou de la chevelure, sans effet de tiraillement, de lourdeur, ou de sensation rigide.

Le problème posé par la présente invention est de fournir des gels homogènes et malléables, apportant à la matière kératinique, notamment aux cheveux, de bonnes propriétés cosmétiques et une fixation élevée, en particulier ces gels permettent d'obtenir une fixation des cheveux très forte et très durable.

De manière surprenante et inattendue, la Demanderesse a constaté que ce problème peut être résolu en utilisant des polyesters sulfoniques ramifiés, à une concentration élevée, c'est-à-dire supérieure à 40 % en poids, dans un milieu alcoolique ou aqueux, par rapport au poids total de la composition.

Cette propriété est d'autant plus surprenante que la réalisation des compositions cosmétiques à base de polyesters sulfoniques ramifiés se révèle difficile compte tenu les propriétés physiques de ce type de polymère.

En effet, la réalisation de telles compositions à partir d'un polyester sulfonique ramifié est difficilement envisageable car sa mise en dispersion ou en solubilisation du polyester est longue, difficile et délicate, dans la mesure ou la matière première est disponible sous forme d'un bloc.

En outre, l'utilisation de polymère de type polyester à forte concentration conduit souvent à des pâtes difficilement préhensibles, dont les performances sont largement insuffisantes en terme de propriétés cosmétique et fixante.

L'invention a pour objet un gel comprenant entre 45 et 58% en poids de polyester sulfonique ramifié, dans un milieu aqueux ou hydro alcoolique, par rapport au poids total du gel.

Selon l'invention, l'expression « *entre 40 et x % en poids de polyester sulfonique ramifié »* exclu la valeur 40 % en poids de polyester sulfonique ramifié.

Avantageusement, dans le milieu hydroalcoolique, l'alcool est choisi parmi les alcools en C1 à C4, et de préférence l'éthanol.

Selon l'invention, il est essentiel que le polyester sulfonique soit ramifié.

Un autre objet de l'invention concerne un procédé de préparation d'un gel, comprenant entre 45 et 58 % en poids de polyester sulfonique ramifié, dans un milieu aqueux ou hydroalcoolique, par rapport au poids total de la composition, caractérisé par le fait qu'il comprend une étape de chauffage, entre 45°C et 120°C, d'une composition comprenant le polyester sulfonique ramifié dans le milieu aqueux.

Selon un mode de mise en oeuvre du procédé selon l'invention, on ajoute à ladite composition, éventuellement après agitation et refroidissement, un alcool en C1 à C4, notamment de l'éthanol, de telle sorte que la concentration en alcool en C1 à C4, notamment en éthanol, dans le gel soit comprise entre 0 à 20%.en poids.

En général, le polyester sulfonique ramifié, mis en oeuvre en tant que produit de départ dans le procédé selon l'invention, se présente, en général, sous la forme de bloc(s) solide(s) concassé(s).

Avantageusement, le procédé selon l'invention comprend le chauffage, entre 45°C et 120°C, d'une composition comprenant, en pourcentage relatif en poids:
- entre 45 et 58 % de polyester sulfonique ramifié,
- entre 42 et 55 % d'eau,
éventuellement, après agitation et refroidissement,on ajoute un alcool en C1 à C4, notamment de l'éthanol, de telle sorte que la concentration en alcool en C1 à C4, notamment en éthanol, dans le gel soit comprise entre 0 à 20%.en poids.

Encore un autre objet de l'invention concerne un procédé de préparation d'une composition cosmétique se présentant sous une forme galénique quelconque, caractérisé par le fait qu'il comprend la mise en oeuvre et le chauffage d'un gel aqueux ou hydroalcoolique, comprenant entre 45 et 58 % en poids de polyester sulfonique ramifié, par rapport au poids total du gel.

Encore un autre objet de l'invention concerne l'utilisation du gel comme produit de fixation et/ou de maintien des cheveux ou pour la fabrication de tels produits.

Avantageusement, la température de transition vitreuse des polyesters sulfoniques est comprise entre -15 et +10°C , de préférence entre -12 et +7 °C, plus avantageusement entre -10 et +5 °C ; Avantageusement, le gel comprend, en concentration en poids par rapport au poids total de la composition, 45 et 58 % en poids de polyester sulfonique ramifié,

De préférence, le polyester sulfonique ramifié est un polymère formé par la polycondensation
a) d'au moins un acide dicarboxylique ne portant pas de fonction sulfonique,
b) d'au moins un diol ou d'un mélange d'un diol et d'une amine,
c) d'au moins un monomère comportant deux fonctions réactives, identiques ou différentes, choisies parmi les groupes hydroxyle, amino et carboxyle, et portant en outre au moins une fonction sulfonique, et
d) d'au moins un monomère comportant au moins trois fonctions réactives, identiques ou différentes, choisies parmi les groupes hydroxyle, amino et carboxyle.

Dans le procédé de préparation du polyester sulfonique ramifié, le (ou les) monomère(s) d) induisent la ramification du polyester sulfonique.

Plus préférentiellement, le polyester sulfonique ramifié contient, en outre, des motifs dérivés de monomères difonctionnels (e) choisis parmi les hydroxyacides carboxyliques et les aminoacides carboxyliques ou un mélange de ceux-ci.

De préférence, le monomère difonctionnel (e) représente jusqu'à 40% en moles de l'ensemble des monomères ; le monomère (c) portant au moins une fonction sulfonique représente de 2 à 15% en moles de l'ensemble des monomères ; le monomère (d) comportant au moins trois fonctions réactives représente de 0.1 et 40 en moles rapportée à l'ensemble de monomères.

Avantageusement, les acides dicarboxyliques ne portant pas de fonction sulfonique formant les motif (a) sont choisis parmi les acides dicarboxyliques aliphatiques, les acides dicarboxyliques alicycliques, les acides dicarbxoyliques aromatiques et les mélanges de tels acides.

Plus avantageusement, les acides dicarboxyliques formant les motifs (a) sont choisi dans le groupe formé par l'acide 1,4-cyclohexanedioïque, l'acide succinique, l'acide glutarique, l'acide adipique, l'acide azélaïque, l'acide sébacique, l'acide fumarique, l'acide maléique, l'acide 1,3-cyclohexanedioïque, l'acide phtalique, l'acide téréphtalique et l'acide isophtalique et un mélange de tels acides.

Avantageusement, les diols formant les motifs (b) sont choisis parmi les alcanediols et les polyalkylènediols et sont choisis parmi l'éthylène glycol, le propylène glycol, le diéthylène glycol, le triéthylène glycol et le polypropylène glycol.

Notamment, les diamines formant les motifs (b) sont choisis parmi les alcanediamines et les poly(oxyalkylène)diamines.

Avantageusement, le monomère (c) portant au moins une fonction sulfonique est choisis parmi les acides dicarboxyliques, les esters d'acides dicarboxyliques, les glycols et d'hydroxyacides, portant tous au moins un groupe sulfonique, sous forme acide et/ou neutralisée, de préférence sous forme neutralisée.

Avantageusement, le monomère multifonctionnel (d) est choisi parmi le triméthyloléthane, le triméthylolpropane, le glycérol, le pentaérythritol, le sorbitol, l'anhydride trimellitique, l'érythritol, le thréitol, le dipentaérythritol, le dianhydride pyromellitique et l'acide diméthylpropionique.

Avantageusement, dans le procédé de préparation du gel selon l'invention, la température est comprise entre 55°C et 100°C, de préférence, comprise entre 70°C et 90°C. Préférentiellement, le chauffage est effectué sous agitation.

Avantageusement, dans le procédé de préparation d'une composition cosmétique se présentant sous une forme galénique quelconque, la température est comprise entre 55°C et 100°C, plus préférentiellement, entre 70°C et 90°C, préférentiellement, le chauffage est effectué sous agitation, et on met en oeuvre le gel aqueux ou hydroalcoolique en dispersion dans un milieu cosmétiquement acceptable.

Le terme *« fonction sulfonique »* des motifs (c) englobe à la fois la fonction acide sulfonique (-SO₃H) et les fonctions salifiées correspondantes obtenues par neutralisation de la fonction acide sulfonique avec une base, par exemple avec un hydroxyde de métal alcalin.

Les polyesters sulfoniques ramifiés peuvent comporter, en plus des quatre type de motifs (a) à (d) décrits ci-dessus, des motifs (e) dérivés de monomères comportant deux fonctions réactives différentes, choisis par exemple parmi les hydroxyacides carboxyliques et les aminoacides carboxyliques ou un mélange de ceux-ci.

Ces motifs (e) peuvent représenter jusqu'à 40% en moles de l'ensemble des monomères (a), (b), (c), (d) et (e).

Bien entendu, les polymères sulfoniques ramifiés utilisé dans la présente invention sont obtenus de préférence à partir d'un mélange de monomères dans lequel le nombre d'équivalents de fonctions acide carboxylique est substantiellement égal au nombre d'équivalents de fonctions hydroxyle et de fonctions amino, éventuellement présentes.

Les polymères sulfoniques utilisés dans le procédé de l'invention sont connus et commercialisés par exemple par la société EASTMAN. Parmi ces polymères, nous préférerons ceux commercialisés sous les dénominations AQ 1045, AQ 1350 et AQ 14000. On peut citer à titre de produit commercial particulièrement préféré le produit vendu sous la dénomination AQ 1350 par la société EASTMAN.

Le procédé de préparation du polyester sulfonique mis en oeuvre selon l'invention est, par exemple, décrit dans le brevet US 5 543 488.

Les compositions cosmétiques préparées selon le procédé de la présente invention peuvent contenir en outre un ou plusieurs solvants additionnels.

Ces solvants peuvent être volatils ou non et englobent notamment l'eau, les alcools aliphatiques ou aromatiques en C₂₋₁₈, les polyols en C₂₋₃₀, les alcanes en C₄₋₁₈, les silicones fluides volatiles ou non-volatiles, linéaires ou cycliques, l'acétone, la méthyléthylcétone, l'acétate de méthyle, l'acétate de butyle, l'acétate d'éthyle, le diméthoxyéthane ou le diéthoxyéthane.

Les solvants additionnels qui se sont avérés particulièrement avantageux pour une utilisation dans les compositions cosmétiques de la présente invention sont les silicones volatiles, en particulier les silicones cycliques volatiles, telles que le décaméthylpentasiloxane (D₅).

Les compositions cosmétiques préparées selon le procédé de la présente invention peuvent contenir en outre un ou plusieurs principes actifs cosmétiques et/ou un ou plusieurs adjuvants de formulation. On peut citer à titre de tels principes actifs et adjuvants les huiles minérales, végétales ou animales, les cires oxyéthylénées ou non, les paraffines, les acides gras, les amides gras, les esters gras, les alcools gras, les agents réducteurs, les agents oxydants, les séquestrants, les agents épaississants, les agents adoucissants, les agents anti-mousse, les agents hydratants, les agents émollients, les agents d'ajustement du pH, les agents plastifiants, les filtres solaires, les colorants directs, les précurseurs de colorants d'oxydation (bases et coupleurs), les pigments, les charges minérales, les argiles, les nacres, les parfums, les agents peptisants, les conservateurs, les agents tensioactifs anioniques, cationiques, non-ioniques, amphotères et zwitterionique, les polymères fixants, les polymères conditionneurs, les protéines, les vitamines.

L'homme du métier veillera à choisir le ou les solvants additionnels, le ou les principes actifs et/ou le ou les adjuvants de formulation de manière à ne pas nuire aux propriétés avantageuses intrinsèques des compositions de la présente invention.

Les compositions préparées selon le procédé de la présente invention peuvent se présenter sous n'importe quelle forme permettant une application aisée ainsi qu'une répartition régulière sur la chevelure. Il peut s'agir par exemple d'une lotion, d'une mousse, d'un gel ou d'une émulsion. Ces compositions peuvent être appliquées selon un mode rincé ou un mode non rincé, de préférence non rincé.

Le procédé de préparation d'une composition se présentant sous une forme galénique quelconque comprend la préparation du gel aqueux ou hydroalcoolique par chauffage, entre 45°C et 120°C, d'une composition comprenant, en pourcentage relatif en poids:
- entre 45 et 58 % de polyester sulfonique ramifié,
- entre 42 et 55 % d'eau,
éventuellement, après agitation et refroidissement, on ajoute un alcool en C1 à C4, notamment de l'éthanol, de telle sorte que la concentration en alcool en C1 à C4, notamment en éthanol, dans le gel soit comprise entre 0 à 20%.en poids.

On peut envisager le conditionnement de ces compositions dans un dispositif permettant l'application par pulvérisation tel qu'un flacon-pompe ou un dispositif aérosol, en présence d'au moins un agent propulseur. Cet agent propulseur est de préférence choisi parmi l'air, le gaz carbonique, l'azote, le diméthyléther, les hydrocarbures halogénés et les mélanges de ceux-ci.

Un autre objet de la présente invention se rapporte à un procédé cosmétique de soin et/ou de protection et/ou de maquillage de la peau, y compris du cuir chevelu, et/ou des fibres kératiniques humaines comme les cheveux, les cils et les sourcils, comprenant l'application d'une composition cosmétique sous forme de gel telle que définie précédemment, sur la peau et/ou lesdites fibres kératiniques humaines. Le soin de la peau peut notamment consister en la réparation ou la prévention de celle-ci contre les méfaits du temps, comme la sécheresse, les rides et ridules, la mollesse. La protection de la peau vise en particulier la protection de cette dernière contre les stimuli extérieurs comme le vent, la pollution, le rayonnement U. V.

Un autre objet de la présente invention se rapporte à un procédé cosmétique de conditionnement des matières kératiniques humaines et notamment de la peau, y compris du cuir chevelu, et/ou des fibres kératiniques humaines, comprenant l'application d'une composition cosmétique telle que définie précédemment, sur ces matières kératiniques.

Encore un autre objet de la présente invention se rapporte à l'utilisation de cette composition sous forme de gel pour la fabrication de compositions cosmétiques destinées au soin et/ou à la protection et/ou au maquillage des matières kératiniques humaines telles que la peau, les cheveux, les cils, les ongles, les lèvres. Plus spécialement, l'invention a encore pour objet l'utilisation de cette composition pour la fabrication d'un produit cosmétique capillaire, en vue de maintenir et/ou de fixer la coiffure.

Dans les produits cosmétiques élaborés à partir du gel initial, la concentration en polyester sulfonique ramifié est comprise entre 0,1 et 39 %, de préférence entre 0,5 et 25 % par rapport au poids total de la composition cosmétique.

Un autre objet de l'invention concerne l'utilisation du gel pour le soin et/la protection et/le maquillage des matières kératiniques humaines telles que la peau, les cheveux, les cils, les ongles, les lèvres.

Ce gel peut également être utilisé dans le domaine de l'industrie du papier, bâtiment, des adhésifs, des copieurs, des produits ménagers, des lessives, du bois, de la métallurgie, de la peinture, du coating, des plastiques, du cuir, de la textile, de l'automobile, de l'aéronautique, de l'électronique, de l'informatique, des produits jetables en papier comme les couches, du packaging, du pétrole, des pansements.

Les exemples ci-après illustrent la présente invention sans en limiter la portée.

Dans les exemples, MA signifie matière active et les pourcentages sont exprimés en poids par rapport au poids total de la composition.

### Exemples:

### 1. Préparation d'un gel concentré en polyester sulfonique ramifié

| | |
|---|---|
| Polyester sulfonique ramifié AQ 1350 ® (Eastman) | 43.5% MA |
| Eau | qsp 100% |

Le procédé de réalisation du gel aqueux d'un polyester sulfonique consiste à disperser l'AQ 1350 à chaud (80°C) dans l'eau.

Cette préparation du polyester sulfonique ramifié facilite la mise en oeuvre industrielle de l'AQ 1350. Le gel résultant peut être utilisé dans la réalisation de compostions cosmétiques aqueuses à base d'AQ 1350.

### 2. Gel hydro alcoolique d'AQ 1350

| | |
|---|---|
| Polyester sulfonique ramifié AQ 1350 (Eastman) | 47.5% MA |
| Ethanol | 15% |
| Eau | qsp 100% |

Le procédé de réalisation du gel hydro alcoolique consiste à disperser l'AQ 1350 sous agitation à chaud (80°C) dans de l'eau. L'ensemble est ensuite refroidi et l'éthanol est ajouté sous agitation.

## Revendications

1. Gel comprenant entre 45 et 58% en poids de polyester sulfonique ramifié, dans un milieu aqueux ou hydro alcoolique, par rapport au poids total du gel.

2. Gel selon la revendication 1, **caractérisé par le fait que** la température de transition vitreuse des polyesters sulfoniques est comprise entre -15 et +10°C.

3. Gel selon la revendication 1 ou 2, **caractérisé par le fait que** le polyester sulfonique ramifié est un polymère formé par la polycondensation
a) d'au moins un acide dicarboxylique ne portant pas de fonction sulfonique,
b) d'au moins un diol ou d'un mélange d'un diol et d'une amine,
c) d'au moins un monomère comportant deux fonctions réactives, identiques ou différentes, choisies parmi les groupes hydroxyle, amino et carboxyle, et portant en outre au moins une fonction sulfonique, et
d) d'au moins un monomère comportant au moins trois fonctions réactives, identiques ou différentes, choisies parmi les groupes hydroxyle, amino et carboxyle.

4. Gel selon l'une quelconque des revendications précédentes, **caractérisé par le fait que** le polyester sulfonique ramifié contient, en outre, des motifs dérivés de monomères difonctionnels (e) choisis parmi les hydroxyacides carboxyliques et les aminoacides carboxyliques ou un mélange de ceux-ci.

5. Gel selon la revendication 4, **caractérisé par le fait que** le monomère difonctionnel (e) représente jusqu'à 40% en moles de l'ensemble des monomères.

6. Gel selon l'une quelconques des revendications 3 à 5, **caractérisé par le fait que** le monomère (c) portant au moins une fonction sulfonique représente de 2 à 15% en moles de l'ensemble des monomères.

7. Gel selon l'une quelconques des revendications 3 à 6, **caractérisé par le fait que** le monomère (d) comportant au moins trois fonctions réactives représente de 0.1 et 40 en moles rapportée à l'ensemble de monomères.

8. Gel selon l'une quelconques des revendications 3 à 7, **caractérisé par le fait que** les acides dicarboxyliques ne portant pas de fonction sulfonique formant les motif (a) sont choisis parmi les acides dicarboxyliques aliphatiques, les acides dicarboxyliques alicycliques, les acides dicarbxoyliques aromatiques et les mélanges de tels acides.

9. Gel selon l'une quelconques des revendications 3 à 8, **caractérisé par le fait que** les acides dicarboxyliques formant les motifs (a) sont choisi dans le groupe formé par l'acide 1,4-cyclohexanedioïque, l'acide succinique, l'acide glutarique, acide adipique, l'acide azélaïque, l'acide sébacique, l'acide fumarique, l'acide maléique, l'acide 1,3-cyclohexanedioïque, l'acide phtalique, l'acide téréphtalique et l'acide isophtalique et un mélange de tels acides.

10. Gel selon l'une quelconques des revendications 3 à 9, **caractérisé par le fait que** les diols formant les motifs (b) sont choisis parmi les alcanediols et les polyalkylènedlols.

11. Gel selon l'une quelconques des revendications 3 à 10, **caractérisé par le fait que** les diols formant les motifs (b) sont choisis parmi l'éthylène glycol, le propylène glycol, le diéthylène glycol, le triéthylène glycol et le polypropylène glycol.

12. Gel selon l'une quelconques des revendications 3 à 11, **caractérisé par le fait que** les diamines formant les motifs (b) sont choisis parmi les alcanediamines et les poly(oxyalkylène)diamines.

13. Gel selon l'une quelconques des revendications 3 à 12, **caractérisé par le fait que** le monomère (c) portant au moins une fonction sulfonique est choisis parmi les acides dicarboxyliques, les esters d'acides dicarboxyliques, les glycols et d'hydroxyacides, portant tous au moins un groupe sulfonique, sous forme acide et/ou neutralisée, de préférence sous forme neutralisée.

14. Gel selon l'une quelconques des revendications 3 à 13, **caractérisé par le fait que** le monomère multifonctionnel (d) est choisi parmi le triméthyloléthane, le triméthylolpropane, le glycérol, le pentaérythritol, le sorbitol, l'anhydride trimellitique, l'érythritol, le thréitol, le dipentaérythritol, le dianhydride pyromellitique et l'acide diméthylpropionique.

15. Procédé de préparation d'un gel selon l'une quelconque des revendications précédentes, **caractérisé par le fait qu'**il comprend une étape de chauffage, entre 45°C et 120°C, d'une composition comprenant le polyester sulfonique ramifié dans le milieu aqueux.

16. Procédé selon la revendication 15, **caractérisé par le fait qu'**on ajoute à ladite composition, éventuellement après agitation et refroidissement, un alcool en C1 à C4, notamment de l'éthanol, de telle sorte que la concentration en alcool en C1 à C4, notamment en éthanol, dans le gel soit comprise entre 0 à 20%.en poids.

17. Procédé selon l'une quelconque des revendications 15 ou 16, **caractérisé par le fait qu'**il comprend le chauffage, entre 45°C et 120°C, d'une composition comprenant, en pourcentage relatif en poids:
- entre 45 et 58 % de polyester sulfonique ramifié,
- entre 42 et 55 % d'eau,
éventuellement après agitation et refroidissement, on ajoute un alcool en C1 à C4, notamment de l'éthanol, de telle sorte que la concentration en alcool en C1 à C4, notamment en éthanol, dans le gel soit comprise entre 0 à 20%.en poids.

18. Procédé selon l'une quelconque des revendications 15 à 17, **caractérisé par le fait que** la température est comprise entre 55°C et 100°C.

19. Procédé selon l'une quelconque des revendications 15 à 18, **caractérisé par le fait que** la température est comprise entre 70°C et 90°C.

20. Procédé selon l'une quelconque des revendications 15 à 19, **caractérisé par le fait que** le chauffage est effectué sous agitation.

21. Procédé selon l'une quelconque des revendications 15 à 20, **caractérisé par le fait que** la température de transition vitreuse des polyesters sulfoniques est comprise entre -15 et +10°C.

22. Procédé de préparation d'une composition cosmétique se présentant sous une forme galénique quelconque, **caractérisé par le fait qu'**il comprend la mise en oeuvre et le chauffage d'un gel aqueux ou hydroalcoolique, comprenant entre 45 et 58 % en poids de polyester sulfonique ramifié, par rapport au poids total du gel.

23. Procédé selon la revendication 22, **caractérisé par le fait qu'**il comprend la préparation du gel aqueux ou hydroalcoolique par chauffage, entre 45°C et 120°C, d'une composition comprenant, en pourcentage relatif en poids:
- entre 45 et 58 % de polyester sulfonique ramifié,
- entre 42 et 55 % d'eau,
éventuellement, après agitation et refroidissement, on ajoute un alcool en C1 à C4, notamment de l'éthanol, de telle sorte que la concentration en alcool en C1 à C4, notamment en éthanol, dans le gel soit comprise entre 0 à 20%.en poids.

24. Procédé selon l'une quelconque des revendications 22 ou 23, **caractérisé par le fait que** la température est comprise entre 55°C et 100°C.

25. Procédé selon l'une quelconque des revendications 22 à 24, **caractérisé par le fait que** la température est comprise entre 70°C et 90°C.

26. Procédé selon l'une quelconque des revendications 22 à 25, **caractérisé par le fait que** le chauffage est effectué sous agitation.

27. Procédé selon quelconque des revendications 22 à 26, **caractérisé par le fait qu'**on met en oeuvre le gel aqueux ou hydroalcoolique en dispersion dans un milieu cosmétiquement acceptable.

28. Utilisation d'un gel selon l'une quelconque des revendications 1 à 14 du gel comme produit de fixation et/ou de maintien des cheveux ou pour la fabrication de tel produit.

29. Utilisation d'un gel selon l'une quelconque des revendications 1 à 14, pour la fabrication de compositions cosmétiques destinées au soin et/ou à la protection et/ou au maquillage des matières kératiniques humaines telles que la peau, les cheveux, les cils, les ongles, les lèvres.

30. Utilisation du gel selon l'une quelconque des revendications 1 à 14, pour le soin et/la protection et/le maquillage des matières kératiniques humaines telles que la peau, les cheveux, les cils, les ongles, les lèvres.

## Claims

1. Gel comprising between 45 and 58% by weight of branched sulfonic polyester, in an aqueous or aqueous/alcoholic medium, with respect to the total weight of the gel.

2. Gel according to Claim 1, **characterized in that** the glass transition temperature of the sulfonic polyesters is between -15 and +10°C.

3. Gel according to Claim 1 or 2, **characterized in that** the branched sulfonic polyester is a polymer formed by the polycondensation
a) of at least one dicarboxylic acid not carrying a sulfonic functional group,
b) of at least one diol or of a mixture of a diol and of an amine,
c) of at least one monomer comprising two reactive functional groups, which are identical or different, chosen from hydroxyl, amino and carboxyl groups and additionally carrying at least one sulfonic functional group, and
d) of at least one monomer comprising at least three reactive functional groups, which are identical or different, chosen from hydroxyl, amino and carboxyl groups.

4. Gel according to any one of the preceding claims, **characterized in that** the branched sulfonic polyester additionally comprises units derived from difunctional monomers (e) chosen from hydroxycarboxylic acids and aminocarboxylic acids or a mixture of these.

5. Gel according to Claim 4, **characterized in that** the difunctional monomer (e) represents up to 40 mol% of the combined monomers.

6. Gel according to any one of Claims 3 to 5, **characterized in that** the monomer (c) carrying at least one sulfonic functional group represents from 2 to 15 mol% of the combined monomers.

7. Gel according to any one of Claims 3 to 6, **characterized in that** the monomer (d) comprising at least three reactive functional groups represents from 0.1 to 40 mol% with respect to the combined monomers.

8. Gel according to any one of Claims 3 to 7, **characterized in that** the dicarboxylic acids not carrying a sulfonic functional group forming the units (a) are chosen from aliphatic dicarboxylic acids, alicyclic dicarboxylic acids, aromatic dicarboxylic acids and mixtures of such acids.

9. Gel according to any one of Claims 3 to 8, **characterized in that** the dicarboxylic acids forming the units (a) are chosen from the group formed by 1,4-cyclohexanedioic acid, succinic acid, glutaric acid, adipic acid, azelaic acid, sebacic acid, fumaric acid, maleic acid, 1,3-cyclohexanedioic acid, phthalic acid, terephthalic acid and isophthalic acid, and a mixture of such acids.

10. Gel according to any one of Claims 3 to 9, **characterized in that** the diols forming the units (b) are chosen from alkanediols and polyalkylenediols.

11. Gel according to any one of Claims 3 to 10, **characterized in that** the diols forming the units (b) are chosen from ethylene glycol, propylene glycol, diethylene glycol, triethylene glycol and polypropylene glycol.

12. Gel according to any one of Claims 3 to 11, **characterized in that** the diamines forming the units (b) are chosen from alkanediamines and poly(oxyalkylene)diamines.

13. Gel according to any one of Claims 3 to 12, **characterized in that** the monomer (c) carrying at least one sulfonic functional group is chosen from dicarboxylic acids, esters of dicarboxylic acids, glycols and hydroxy acids all carrying at least one sulfonic group in the acid and/or neutralized form, preferably in the neutralized form.

14. Gel according to any one of Claims 3 to 13, **characterized in that** the polyfunctional monomer (d) is chosen from trimethylolethane, trimethylolpropane, glycerol, pentaerythritol, sorbitol, trimellitic anhydride, erythritol, threitol, dipentaerythritol, pyromellitic dianhydride and dimethylpropionic acid.

15. Process for the preparation of a gel according to any one of the preceding claims, **characterized in that** it comprises a stage of heating, between 45°C and 120°C, a composition comprising the branched sulfonic polyester in the aqueous medium.

16. Process according to Claim 15, **characterized in that** a C₁ to C₄ alcohol, in particular ethanol, is added to said composition, optionally after stirring and cooling, so that the concentration of C₁ to C₄ alcohol, in particular of ethanol, in the gel is between 0 and 20% by weight.

17. Process according to either one of Claims 15 and 16, **characterized in that** it comprises heating, between 45°C and 120°C, a composition comprising, as relative percentage by weight:
- between 45 and 58% of branched sulfonic polyester,
- between 42 and 55% of water,
optionally, after stirring and cooling, a C₁ to C₄ alcohol, in particular ethanol, is added so that the concentration of C₁ to C₄ alcohol, in particular of ethanol, in the gel is between 0 and 20% by weight.

18. Process according to any one of Claims 15 to 17, **characterized in that** the temperature is between 55°C and 100°C.

19. Process according to any one of Claims 15 to 18, **characterized in that** the temperature is between 70°C and 90°C.

20. Process according to any one of Claims 15 to 19, **characterized in that** the heating is carried out with stirring.

21. Process according to any one of Claims 15 to 20, **characterized in that** the glass transition temperature of the sulfonic polyesters is between -15 and +10°C.

22. Process for the preparation of a cosmetic composition which is provided in any formulation form, **characterized in that** it comprises the use and the heating of an aqueous or aqueous/alcoholic gel comprising between 45 and 58% by weight of branched sulfonic polyester, with respect to the total weight of the gel.

23. Process according to Claim 22, **characterized in that** it comprises the preparation of the aqueous or aqueous/alcoholic gel by heating, between 45°C and 120°C, a composition comprising, as relative percentage by weight:
- between 45 and 58% of branched sulfonic polyester,
- between 42 and 55% of water,
optionally, after stirring and cooling, a C₁ to C₄ alcohol, in particular ethanol, is added so that the concentration of C₁ to C₄ alcohol, in particular of ethanol, in the gel is between 0 and 20% by weight.

24. Process according to either one of Claims 22 and 23, **characterized in that** the temperature is between 55°C and 100°C.

25. Process according to any one of Claims 22 to 24, **characterized in that** the temperature is between 70°C and 90°C.

26. Process according to any one of Claims 22 to 25, **characterized in that** the heating is carried out with stirring.

27. Process according to any one of Claims 22 to 26, **characterized in that** the aqueous or aqueous/alcoholic gel is employed in dispersion in a cosmetically acceptable medium.

28. Use of a gel according to any one of Claims 1 to 14 as product for the fixing and/or form retention of the hair or for the manufacture of such product.

29. Use of a gel according to any one of Claims 1 to 14 for the manufacture of cosmetic compositions intended for caring for and/or protecting and/or making up human keratinous substances, such as the skin, hair, eyelashes, nails or lips.

30. Use of the gel according to any one of Claims 1 to 14 for caring for and/or protecting and/or making up human keratinous substances, such as the skin, hair, eyelashes, nails or lips.

## Patentansprüche

1. Gel, umfassend zwischen 45 und 58 Gew.-% verzweigten Sulfopolyester in einem wässrigen oder wässrig-alkoholischen Medium, bezogen auf das Gesamtgewicht des Gels.

2. Gel nach Anspruch 1, **dadurch gekennzeichnet, dass** die Glasübergangstemperatur der Sulfopolyester zwischen -15 und +10°C liegt.

3. Gel nach Anspruch 1 oder 2, **dadurch gekennzeichnet, dass** es sich bei dem verzweigten Sulfopolyester um ein durch Polykondensation von
a) mindestens einer Dicarbonsäure, die keine Sulfofunktion trägt,
b) mindestens einem Diol oder einer Mischung von einem Diol und einem Amin,
c) mindestens einem Monomer, das zwei gleiche oder verschiedene reaktive Funktionen, die unter Hydroxyl-, Amino- und Carboxylgruppen ausgewählt sind, enthält und außerdem mindestens eine Sulfofunktion trägt, und
d) mindestens einem Monomer, das mindestens drei gleiche oder verschiedene reaktive Funktionen, die unter Hydroxyl-, Amino- und Carboxylgruppen ausgewählt sind, enthält,
gebildetes Polymer handelt.

4. Gel nach einem der vorhergehenden Ansprüche, **dadurch gekennzeichnet, dass** der verzweigte Sulfopolyester außerdem Einheiten enthält, die sich von difunktionellen Monomeren (e), die unter Hydroxycarbonsäuren und Aminocarbonsäuren oder einer Mischung davon ausgewählt sind, ableiten.

5. Gel nach Anspruch 4, **dadurch gekennzeichnet, dass** das difunktionelle Monomer (e) bis zu 40 Mol-% der gesamten Monomere ausmacht.

6. Gel nach einem der Ansprüche 3 bis 5, **dadurch gekennzeichnet, dass** das Monomer (c), das mindestens eine Sulfofunktion trägt, 2 bis 15 Mol-% der gesamten Monomere ausmacht.

7. Gel nach einem der Ansprüche 3 bis 6, **dadurch gekennzeichnet, dass** das Monomer (d), das mindestens drei reaktive Funktionen enthält, 0,1 bis 40 Mol-% der gesamten Monomere ausmacht.

8. Gel nach einem der Ansprüche 3 bis 7, **dadurch gekennzeichnet, dass** die die Einheiten (a) bildenden Dicarbonsäuren, die keine Sulfofunktion tragen, unter aliphatischen Dicarbonsäuren, alicyclischen Dicarbonsäuren, aromatischen Dicarbonsäuren und Mischungen derartiger Säuren ausgewählt sind.

9. Gel nach einem der Ansprüche 3 bis 8, **dadurch gekennzeichnet, dass** die die Einheiten (a) bildenden Dicarbonsäuren aus der Gruppe bestehend aus 1,4-Cyclohexandisäure, Bernsteinsäure, Glutarsäure, Adipinsäure, Azelainsäure, Sebacinsäure, Fumarsäure, Maleinsäure, 1,3-Cyclohexandisäure, Phthalsäure, Terephthalsäure und Isophthalsäure und einer Mischung derartiger Säuren ausgewählt sind.

10. Gel nach einem der Ansprüche 3 bis 9, **dadurch gekennzeichnet, dass** die die Einheiten (b) bildenden Diole unter Alkandiolen und Polyalkylendiolen ausgewählt sind.

11. Gel nach einem der Ansprüche 3 bis 10, **dadurch gekennzeichnet, dass** die die Einheiten (b) bildenden Diole unter Ethylenglykol, Propylenglykol, Diethylenglykol, Triethylenglykol und Polypropylenglykol ausgewählt sind.

12. Gel nach einem der Ansprüche 3 bis 11, **dadurch gekennzeichnet, dass** die die Einheiten (b) bildenden Diamine unter Alkandiaminen und Poly(oxyalkylen)diaminen ausgewählt sind.

13. Gel nach einem der Ansprüche 3 bis 12, **dadurch gekennzeichnet, dass** das Monomer (c), das mindestens eine Sulfofunktion trägt, unter Dicarbonsäuren, Dicarbonsäureestern, Glykolen und Hydroxysäuren, die alle mindestens eine Sulfogruppe in saurer und/oder neutralisierter Form, vorzugsweise in neutralisierter Form, tragen, ausgewählt ist.

14. Gel nach einem der Ansprüche 3 bis 13, **dadurch gekennzeichnet, dass** das multifunktionelle Monomer (d) unter Trimethylolethan, Trimethylolpropan, Glycerin, Pentaerythrit, Sorbit, Trimellitsäureanhydrid, Erythrit, Threit, Dipentaerythrit, Pyromellitsäuredianhydrid und Dimethylpropionsäure ausgewählt ist.

15. Verfahren zur Herstellung eines Gels nach einem der vorhergehenden Ansprüche, **dadurch gekennzeichnet, dass** es einen Schritt des Erhitzens einer Zusammensetzung, die den verzweigten Sulfopolyester umfasst, in dem wässrigen Medium auf eine Temperatur zwischen 45°C und 120°C umfasst.

16. Verfahren nach Anspruch 15, **dadurch gekennzeichnet, dass** man, gegebenenfalls nach Rühren und Abkühlen, einen C1- bis C4-Alkohol, insbesondere Ethanol, derart zu der Zusammensetzung gibt, dass die Konzentration an C1- bis C4-Alkohol, insbesondere Ethanol, in dem Gel zwischen 0 und 20 Gew.-% liegt.

17. Verfahren nach einem der Ansprüche 15 oder 16, **dadurch gekennzeichnet, dass** man eine Zusammensetzung, die in Gewichtsprozent:
- zwischen 45 und 58% verzweigten Sulfopolyester,
- zwischen 42 und 55 Gew.-% Wasser umfasst, auf eine Temperatur zwischen 45°C und 120°C erhitzt
und gegebenenfalls nach Rühren und Abkühlen einen C1- bis C4-Alkohol, insbesondere Ethanol, derart zugibt, dass die Konzentration an C1- bis C4-Alkohol, insbesondere Ethanol, in dem Gel zwischen 0 und 20 Gew.-% liegt.

18. Verfahren nach einem der Ansprüche 15 bis 17, **dadurch gekennzeichnet, dass** die Temperatur zwischen 55°C und 100°C liegt.

19. Verfahren nach einem der Ansprüche 15 bis 18, **dadurch gekennzeichnet, dass** die Temperatur zwischen 70°C und 90°C liegt.

20. Verfahren nach einem der Ansprüche 15 bis 19, **dadurch gekennzeichnet, dass** man das Erhitzen unter Rühren durchführt.

21. Verfahren nach einem der Ansprüche 15 bis 20, **dadurch gekennzeichnet, dass** die Glasübergangstemperatur der Sulfopolyester zwischen -15 und +10°C liegt.

22. Verfahren zur Herstellung einer kosmetischen Zusammensetzung, die in einer galenischen Form vorliegt, **dadurch gekennzeichnet, dass** man ein wässriges oder wässrig-alkoholisches Gel, das zwischen 45 und 58 Gew.-% verzweigten Sulfopolyester, bezogen auf das Gesamtgewicht des Gels, umfasst, verwendet und erhitzt.

23. Verfahren nach Anspruch 22, **dadurch gekennzeichnet, dass** es die Herstellung des wässrigen oder wässrig-alkoholischen Gels umfasst, indem man eine Zusammensetzung, die in Gewichtsprozent:
- zwischen 45 und 58% verzweigten Sulfopolyester,
- zwischen 42 und 55 Gew.-% Wasser umfasst, auf eine Temperatur zwischen 45°C und 120°C erhitzt
und gegebenenfalls nach Rühren und Abkühlen einen C1- bis C4-Alkohol, insbesondere Ethanol, derart zugibt, dass die Konzentration an C1- bis C4-Alkohol, insbesondere Ethanol, in dem Gel zwischen 0 und 20 Gew.-% liegt.

24. Verfahren nach einem der Ansprüche 22 oder 23, **dadurch gekennzeichnet, dass** die Temperatur zwischen 55°C und 100°C liegt.

25. Verfahren nach einem der Ansprüche 22 bis 24, **dadurch gekennzeichnet, dass** die Temperatur zwischen 70°C und 90°C liegt.

26. Verfahren nach einem der Ansprüche 22 bis 25, **dadurch gekennzeichnet, dass** man das Erhitzen unter Rühren durchführt.

27. Verfahren nach einem der Ansprüche 22 bis 26, **dadurch gekennzeichnet, dass** man das wässrige oder wässrig-alkoholische Gel als Dispersion in einem kosmetisch unbedenklichen Medium verwendet.

28. Verwendung eines Gels nach einem der Ansprüche 1 bis 14 als Produkt zum Fixieren und/oder Haltgeben der Haare oder zur Herstellung eines derartigen Produkts.

29. Verwendung eines Gels nach einem der Ansprüche 1 bis 14 zur Herstellung von kosmetischen Zusammensetzungen für die Pflege und/oder den Schutz und/oder als Makeup von menschlichen Keratinmaterialien wie der Haut, der Haare, der Wimpern, der Nägel oder der Lippen.

30. Verwendung eines Gels nach einem der Ansprüche 1 bis 14 zur Pflege und/oder zum Schutz und/oder als Makeup von menschlichen Keratinmaterialien wie der Haut, der Haare, der Wimpern, der Nägel oder der Lippen.
